# EUROPEAN PATENT SPECIFICATION

(11) **EP 1 324 782 B1**
(45) Date of publication and mention of the grant of the patent: **11.08.2004**
(21) Application number: 01974306.1
(22) Date of filing: 26.09.2001
(51) Int. Cl.: A61L 12/12, A61K 9/00

(54) **STABILIZED OPHTHALMIC HYDROGEN PEROXIDE SOLUTIONS**
STABILISIERTE OPHTHALMISCHE WASSERSTOFFPEROXIDLÖSUNG
SOLUTIONS OPHTHALMIQUES STABILISEES DE PEROXYDE D'HYDROGENE

(30) Priority: 28.09.2000 US 236251 P
(43) Date of publication of application: 09.07.2003
(73) Proprietor: Novartis AG, 4056 Basel (CH); Novartis Pharma GmbH, 1230 Wien (AT)
(72) Inventor: TSAO, Fu-Pao, Mark, Lawrenceville, GA 30243 (US)
(74) Representative: Gros, Florent
(86) International application number: PCT/EP2001/011177
(87) International publication number: WO 2002/026277

(56) References cited:
- EP-A- 0 142 642
- EP-A- 0 219 220
- US-A- 4 568 517
- US-A- 5 225 055
- US-A- 5 725 887

## Description

The present invention relates to stabilized aqueous solutions that contain trace quantities of hydrogen peroxide. More particularly, this invention relates to the use of certain organophophorous compounds as stabilizers for such solutions at high pH. These biocompatible compositions are especially useful in buffered saline for eye care solutions.

Contact lenses accumulate dirt, proteinaceous matter, and microorganisms, all of which can affect the health of the eye if allowed to accumulate on the lens. Therefore the lenses must be cleaned and disinfected regularly and preferably daily. Hydrogen peroxide is recognized as a safe and efficacious disinfectant for contact lenses and contact lens disinfecting solutions that contain hydrogen peroxide are well known.

One drawback to the use dilute hydrogen peroxide solutions, however, is that without the use of a stabilizer, or a combination of stabilizers, the aqueous peroxide solutions characteristically decompose over a relatively short time period. The actual rate at which such dilute hydrogen peroxide solutions decompose will, of course, be dependent upon such factors as pH and the presence of trace amounts of various metal impurities, such as copper or chromium, which may act to catalytically decompose the same. Moreover, at moderately elevated temperatures, the rate of decomposition of such dilute aqueous hydrogen peroxide solutions is greatly accelerated. PCT patent WO98/04496 discloses stabilized and buffered solutions of hydrogen peroxide for contact lens disinfection in which the solution is maintained in the pH range of about 5.0 to 6.5 by the use of certain phosphonic acids and derivatives thereof. Also, British Patent No. 1,500,707 discloses a contact lens sterilizing solution using hydrogen peroxide with 200 to 2000 ppm of a phosphate [pyrophosphate] stabilizer at a pH of 4.5. However, the disinfection is not carried out at a pH consistent with the ocular environment, since the pH must be to elevated to around 7 to 8 for the solution to be physiologically acceptable.

U.S. patent No. 5,725,887 discloses a preservative for ophthalmic solutions having a low hydrogen peroxide concentration in the presence of various phosphonic acid stabilizers. However, the stabilizing effect of these compositions requires that these solutions be maintained at a pH less than 8.0 and most preferably between about 6.5 and 7.5

Therefore, there exists a need for preserved contact lens care solutions with improved stability at the higher pH required for ophthalmic compatibility, and that such stabilized solutions contain only trace quantities of hydrogen peroxide or peroxy compounds that generate hydrogen peroxide. It must be recognized that all components of such systems must be compatible with the other ingredients common to ophthalmic solutions and with a variety of ophthalmic medicinal agents.

An objective of the present invention is to provide a means for stabilizing contact lens care solutions that contain low levels of hydrogen peroxide. Such solutions are to be free of materials that are not ocularly compatible and such solutions are to be stable at pH greater than 8. A further objective of the present invention is to provide a means for increasing the shelf-life of ophthalmic solutions which contain hydrogen peroxide and ocularly compatible components. A still further objective of the present invention is to provide preserved ophthalmic drug formulations that are stable and useable at pH greater than 8. These objectives are realized in the present invention through the use of certain ocularly compatible stabilizers that reduce the decomposition rate of the low levels of hydrogen peroxide present in such solutions.

Another advantage of using trace amounts of hydrogen peroxide in the ophthalmic solutions of the present invention is that the low concentration of hydrogen peroxide, especially when concentrations are less than 100 ppm, is destroyed when it comes in contact with the eye. For example, enzymes existing in the eye tissue will cause the breakdown of the hydrogen peroxide into water and oxygen. As a result, the solution, upon application, becomes preservative free and greatly minimizes adverse reactions. Thus, the problems associated with other preservatives, such as the inability to break down innocuous compounds, are eliminated.

One embodiment of the present invention relates to a preserved ophthalmic aqueous solution comprising:
(a) a source of hydrogen peroxide in an amount sufficient to provide hydrogen peroxide in an amount from 2 ppm to 100 ppm ; and
(b) one or more hydrogen peroxide stabilizers selected from the group consisting of
   (a) compounds of the formula 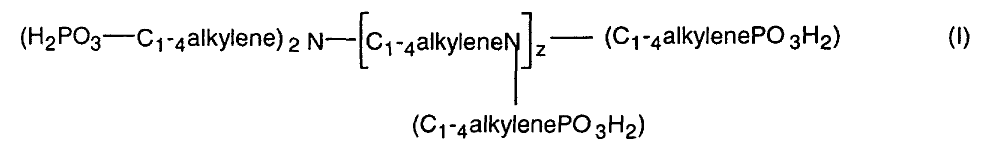 wherein z is an integer from 0-3, and water-soluble salts thereof; and
   (b) compounds of the formula 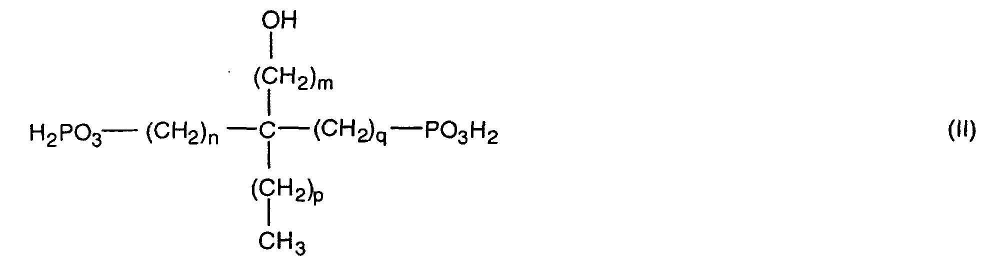 wherein each of n, m, p, and q is independently 0-4, and water-soluble salts thereof, in an amount sufficient to stabilize the resultant hydrogen peroxide; and
(c) said aqueous solution having a pH of between above 8.0 and 10.5.
The solutions of the invention are useful as ophthalmic solutions, which are stabilized above pH 8 by the addition of chelating stabilizers. A surprising aspect of this invention is the clinically observed comfort to the eye of some ophthalmic solutions with pH as high as about 9.5. This demonstrates that stablized ophthalmic solutions preserved with trace amounts of hydrogen peroxide at pH significantly greater than 8 are suitable for use in the human eye. Furthermore the high pH of these solutions allows for the inclusion therein of acid sensitive components such as sodium bicarbonate. Inclusion of such components is not possible in the lower pH solutions of the prior art.

Trace amounts of peroxy compounds stabilized with a hydrogen peroxide stabilizer, especially diethylene triamine penta(methylene phosphonic acid) or 1-hydroxyethylidene-1,1-diphosphonic acid may be utilized as a preservative for drugs, eyewashes, or other solutions containing an active ingredient designed to be used in the ocular environment. The preservative according to the present invention may be used in the ocular environment. Furthermore, the preservative according to the present invention may be used in any ophthalmic solution as long as the active ingredient in that solution is compatible with trace amounts of the peroxy compounds. Also, virtually any peroxy compound may be used so long as it is hydrolyzed in water to produce hydrogen peroxide. Examples of such sources of hydrogen peroxide, which provide an effective resultant amount of hydrogen peroxide, include sodium perborate decahydrate, sodium peroxide and urea peroxide. It has been found that peracetic acid, an organic peroxy compound, cannot be stabilized utilizing the present system. Hydrogen peroxide concentrations from about 2 ppm to about 1000 ppm are useful in the present invention. Therefore, peroxy compounds that generate hydrogen peroxide from about 2 ppm to about 1000 ppm are useful in the present invention. More preferably, the concentration of hydrogen peroxide from is about 10 ppm to about 1000 ppm.

Highly preferred within the stabilizers of formula I are compounds wherein z is 2 and compounds wherein each C₁₋₄ alkylene group is C₁ or C₂. Most preferred within formula I is diethylene triamine penta(methylene-phosphonic acid) and the physiologically compatible water-soluble salts thereof, marketed by Solutia, Inc. (formerly Monsanto) under the name Dequest™ 2060. Highly preferred within the stabilizers of formula II are compounds wherein n, m, p and q are each 0 or 1, most preferably zero, or a physiologically compatible water-soluble salt thereof. Such a compound is marketed by Solutia, Inc. (formerly Monsanto) under the name Dequest™ 2010.

Physiologically compatible salts of the compounds of formulae I and II include, for example, water soluble salts with conventional pharmaceutically acceptable cationic moieties, including the alkali metal, e.g. sodium, potassium, alkaline earth metal, e.g. calcium, ammonium and amine cations. Suitable amine salts include, for example, mono-, di-, and tri-lower alkyl amines, e.g. methylamine, ethylamine, diethylamine, triethylamine, dimethylamine, trimethylamine, propylamine, and the like; and mono-, di-, and tri-lower hydroxyalkyl amines, e.g. ethanolamine, diethanolamine, triethanolamine, glucamine, 2-hydroxypropylamine, and the like. By "lower" in the context of an alkyl group is meant an alkyl group having up to 6 carbon atoms, preferably up to 4 carbon atoms. If desired, additional conventional stabilizers may be employed in conjunction with those of formulae I or II or combinations thereof in accordance with the present invention. Suitable conventional stabilizers include: water soluble stannates, such as an alkali metal or ammonium stannate, for example sodium stannate, alone or in combination with a water soluble phosphate, polyphosphate or etaphosphate salt, such as an alkali metal or ammonium salt thereof; or an amino polycarboxylic acid chelating agent, such as ethylene diamine tetraacetic acid, nitrilo triacetic acid or a water soluble salt thereof, such as an alkali metal or ammonium salt, especially the sodium salt, or mixtures thereof.

The above stabilizers can be used in almost all situations previously mentioned to which the invention is applicable. However, when the solution is to come in contact with a hydrogel soft contact lens, stannate stabilizers are to be avoided as they tend to "cloud" the lens material. Preferably, the concentration of the stabilizer of formula I or salt thereof is present in the stabilized composition in an amount between about 0.006 and about 0.02% by weight of the composition, and most preferably between about 0.006 and about 0.0120% by weight of the composition. The stabilizer of formula II is present per 100 g of solution in an amount of at least about 0.024 m mole (50 ppm), preferably 0.039 m mole (80 ppm) up to about 0.34 m mole (700 ppm) more preferably 0.049 m mole (100 ppm) up to about 0.29 m mole (600 ppm), most preferably 0.073 m mole (150 ppm) to about 0.19 m mole (400 ppm). The amounts in parentheses are for Dequest™ 2010 which has a molecular weight of 206. Other stabilizers of formula II should be present in molar equivalents thereto. The diethylene triamine penta(methylene phosphonic acid) has a molecular weight of 206. Other stabilizers of formula II should be present in molar equivalents thereto. It is most important that any stabilizer chosen be employed in a physiologically tolerable concentration.

The pH of the stabilized hydrogen peroxide solution is between above 8.0 and 10.5, preferably is between above 8.0 and 9.5. The pH can be adjusted as desired by incorporation of suitable amounts of acid or base that is physiologically tolerable in the amounts employed, e.g. hydrochloric acid and sodium hydroxide. The pH of the stabilized solution presents an advantage over the prior art since the pH of most existing ophthalmic solutions containing hydrogen peroxide is relatively low, e.g. less than 7. It has been observed clinically that of some ophthalmic solutions of this invention with pH above 8.0 exhibit a high degree of comfort to the eye. Therefore a hydrogen peroxide preserved lens care solution which has stability at pH >8 is highly desirable.

The pH values of some commercially available hydrogen peroxide products for contact lenses are listed as follows:

| Name of the Product | pH | wt. % H₂O₂ |
|---|---|---|
| AOSept™ (CIBA Vision) | 6.3 - 6.6 | 3.3-3.5 |
| Lensept™ (CIBA Vision) | 3.98 | 3.4 |
| Oxysept™ (Allergan) | 3.28 | 3.3 |
| Mirasept™ (Coopervision) | 3.96 | 3.6 |
| Quiksept™ (Bausch & Lomb) | 3.57 | 3.5 |
| Puresept ™ (Abbott Labs) | 3.83 | 3.4 |
| Softmate II™ (Barnes Hind) | 3.5 - 3.6 | 3.5 |

Trace amounts of peroxy compounds stabilized with a hydrogen peroxide stabilizer, especially diethylene triamine penta(methylene phosphonic acid) or salts thereof or 1-hydroxyethylidene-1,1-diphosphonic acid or salts thereof may be utilized as a preservative for drugs, eyewashes, or other solutions containing an active ingredient designed to be used in the ocular environment.

The full scope of the present invention includes solutions containing medicinally active ophthalmic agents as well as solutions that are free of containing medicinally active ophthalmic agents. The former group of solutions contain at least one medicinal agent for application directly to the eye. The latter group includes, but is not limited to, solutions such as preserved saline, preserved contact lens cleaning solutions, preserved contact lens stabilizing solutions, preserved wetting solutions, and preserved lubricating solutions.

The preservative according to the present invention may be used in any ophthalmic solution as long as the active ingredient in that solution is compatible with trace amounts of the peroxy compounds. It is believed that most compounds, when preserved by the systems of the present invention, are compatible with the trace amounts of hydrogen peroxide employed. The following is a non-exhaustive, non-limiting, illustrative list of active ingredients and excipients that are compatible with the preservative according to the present invention: atropine, homatropine, cyclopentolate, tropicamide, lachesine, dibutoline, oxyphenonium, eucatropine, ephedrine, carbachol, methacholine, pilocarpine hydrochloride, isoflurophate, physostigmine, neostigmine, lignocaine, cocaine, acetylcholine chloride, antazoline phosphate, betaxolol hydrochloride, demecarium bromide, dipivefrin hydrochloride, erythromycin, gentamicin sulfate, homatropine hydrobromide, idoxuridine, isosorbide, lanolin, naphazoline hydrochloride, neomycin sulfate, pheniramine maleate, polysorbate gelatin (Tween), pyrilamine maleate, scopolamine hydrobromide, hyaluronic acid, sodium hyaluronate, tetracaine hydrochloride, oxmetazolin, tetrahydrozoline hydrochloride, diclofenac sodium, dextran, carteolol, sulfanilamide, procaine, proparacaine hydrochloride, sulfisoxazole disolamine, indomethacin, clonidine, corynanthine, arachidonic acid, linoleic acid, H-thymidine and 3H-thymidine, inositol triphosphate, inositol phosphates, phosphatidylinositol and phosphatidylinositol phosphates.

Excipients which are compatible with the present invention include, but are not limited to: polysorbate gelatin (Tween), dextran, linolin, inositol phosphates, alkylsulfosuccinates, Sulfosuccinamate, alkyl silicone sulfosuccinates, alkylpolyether carboxylates, alkylaryl polyethoxylamines, alkylarylsulfonates, alpha olefin sulfonates, alkyl surfates, alkyl ether sulfates, alkanol amides and alkamides, alkylamphoterics, amphoterics based on alkyl imidazoline, betaines, alkylaminopropionates, alkyliminodipropionates, alkylamphoglycinates, alkylamphocarboxyglycinates, alkylamphocarboxypropinates, alkylamphopropionates, alkylamidopropylhydroxysultaines, alkyletherhydroxypropylsultaines, alkylamphopropylsulfonates, quaternary ammonium polymers, quaternary ammonium halides, polyacrylamide, polyacrylates, polyvinyl pyrrolidone, polyvinyl alcohol, alkyl alcohol ethoxylates, hydroxyalkylcelluloses, alkylamidopropyl PG-dimonium chloride phosphates, alkylampho PG-glycinate phosphates, glyceryl monoalkylates, sorbitan alkylates (span), pluronics, tetronics, sodium alkyl sulfates, sodium butoxyethoxy acetate, phosphate esters, glycosides, polyglycosides, mannitol, sorbitol, polyoxyethylene alkyl ethers, guar gum, sodium hyaluronate, polyoxyl 40 stearate, and polyoxyalkylene dimethylpolysiloxane.

However, compounds having non-hindered hydroxyl groups attached to an aromatic ring, such as ketones and alcohols, or having a mercapto group, thioether, acetamido group, or aldehyde group will typically not be peroxide compatible. Such compounds believed non-compatible with trace stabilized hydrogen peroxide include: noradrenaline, adrenaline, phenylephrine hydrochloride, amethocaine, oxybuprocaine, proxymethacaine, cromolyn sodium, benoxinate hydrochloride, chloramphenicol, chlortetracycline hydrochloride, dexamethasone, dichlorphenamide, echotiophate iodide, epinephrine bitartrate, fluorometholone, gramicidin, hydrocortisone, methazolamide, natamycin, prednisolone acetate, sulfacetamide (N -acetylsulfanilamide), tetracycline hydrochloride and timolol maleate.

Also, there may be present in the stabilized hydrogen peroxide solution according to the present invention one or more conventional, substantially inert, physiologically acceptable tonicity enhancing agents. Suitable such agents include, for example, alkali metal halides, phosphates, hydrogen phosphate, and borates. Preferred are sodium chloride, sodium phosphate monobasic and sodium phosphate dibasic. The function of such tonicity enhancing agents is to assure approximate physiologic tonicity to the solution which is instilled in the eye or to help assure such tonicity upon dilution if dilution is necessary prior to contact with the eye due to peroxide content as indicated above.

Preferably sufficient tonicity enhancing agents are present in the solution so that it is substantially isotonic or, such that, upon decomposition or dilution of the hydrogen peroxide therein, the resulting solution is substantially isotonic, e.g. substantially equivalent in tonicity to a 0.9% by weight aqueous sodium chloride solution. The tonicity of common ocular solutions is about 260 -300 milliOsmoles / L. A further optional ingredient is a thickener also known as a viscosity modifying agent or enhancing agent. Any of the substances known in these categories which are ocularly acceptable can be used. Typical suitable thickeners include, inter alia, polyvinyl alcohol, hydroxy ethylcellulose, etc. Thickeners may be present in any amount up to an amount sufficient to raise the overall solution viscosity to about 1000 cps, preferably to not more than 100 cps.

In general, the stabilized hydrogen peroxide solutions of the present invention are characterized by their extraordinary stability, even under the conditions of heat sterilization

in an autoclave, for example 121 °C for 30 min. Thus, the shelf life of these compositions is enhanced. Moreover, the present compositions are characterized by physiological tolerability subsequent to hydrogen peroxide decomposition.
Formulation of the solutions of the invention can be made in any conventional manner. For example, all of the components other than the hydrogen peroxide and water can be placed in a container and fresh, preferably concentrated, hydrogen peroxide added thereto with mixing. Alternatively the dry components can be rubbed up with a small portion of liquid stabilizer, then the remainder of the stabilizer added, followed by the hydrogen peroxide, and most of the water. The viscosity enhancing agent, i.e. thickener, can then be added or the formed solution can be added to the thickener. One of ordinary skill in the art will be aware of numerous variations in the manner of formulating the solutions of the invention.
When it is desirable to neutralize the peroxide activity, any known means, such as rinsing, contacting the solution with platinum, catalase enzymes, or any other substance known to decompose hydrogen peroxide, will suffice. Additional physiological compatible peroxide neutralizing agents include reducing agent such as pyruvic acid and suitable salts thereof such as the sodium salt.

It should be emphasized that the present invention is applicable in the field of ophthalmic device disinfection and preservation provided only that the material treated is not adversely affected by the composition components.

The following examples illustrate the present invention. All parts and percentages are by weight unless otherwise indicated.

EXAMPLE 1: A solution is prepared by dissolving the following ingredients in purified water at the concentrations indicated: carboxymethyl cellulose, 0.25%; magnesium chloride hexahydrate, 0.006%; calcium chloride dehydrate, .0085%; boric acid, 0.17%; sodium chloride, 0.29%; potassium chloride, 0.097%; sodium bicarbonate, 0.145%; sodium borate, 0.001%; sodium phosphate monobasic monohydrate, 0.0074%; diethylene triamine penta(methylene-phosphonic acid) [Dequest™ 2060], 60ppm; and sodium perborate, 0.027%. The solution is adjusted to pH 6.8 by bubbling carbon dioxide gas through the solution. The tonicity of the final solution is 187 mOsm/kg. This solution is stored at room temperature in a 25 ml low density polyethylene (LDPE) dropper bottle. The concentrations of hydrogen peroxide vs. time and pH vs. time are shown in the table below. Hydrogen peroxide concentrations are determined by a standard iodimetric titration.

**TABLE I**

| time in months | pH | conc. of hydrogen peroxide (ppm) |
|---|---|---|
| 0 ^{(a)} | 6.80 | 59 |
| 0 ^{(b)} | 7.29 | NA |
| 26 | 8.37 | >30 |
| 30 | 8.17 | >30 |

| | | |
|---|---|---|
| ^{(a)} = as prepared | | |
| ^{(b)} = after autoclave sterilization for 30 min at 131°C | | |

The data presented in Table I clearly demonstrate that, in this composition, the low levels of hydrogen peroxide in aqueous solution with a pH above 8 has good stability for at least 30 months. This concentration of hydrogen peroxide equal to or greater than 10 ppm easily meets the USP requirement for a preservative.

EXAMPLE 2: This example presents results of a clinical study in which a typical ophthalmic saline solutions is prepared with pH ranging for 7.50 to 9.42 are evaluated for comfort in the eyes. This solution is typical of a lubricating solution used by individuals who suffer from the condition known as "dry eye".

Several aqueous solutions are prepared containing 0.25% CMC (carboxymethyl cellulose), 0.2934 % NaCl, 0.0966% KCI, 0.1451% NaHCO₃, 0.0066% MgCl₂-6H₂O, 0.0085% CaCl₂-2H₂O, 0.074% NaH₂PO₄, 0.1658% boric acid, 0.0001 % NaBO₃. By the addition of dilute NaOH the pH of individual solutions are adjusted to the values shown in Table II.

**TABLE II**

| solution | pH |
|---|---|
| A | 7.50 |
| B | 7.95 |
| C | 8.50 |
| D | 9.00 |
| E | 9.42 |

Drops of each the solutions are applied to both eyes of clinical patients and after about 5 minutes the comfort level of the respective solutions in the eyes is verbally reported by the patients and recorded. The results are presented in Table III.

**TABLE III**

| test # | solution in left eye | solution in right eye | solution in left eye | solution in right eye | comfort level |
|---|---|---|---|---|---|
| 1 | A | A | A | A | comfortable |
| 2 | B | A | A | B | same as test 1 |
| 3 | C | A | A | C | same as test 1 |
| 4 | D | A | A | D | same as test 1 |
| 5 | E | A | A | E | similar to test except more lubricious |
| 6 | A | E | E | A | soln. E more comfortable than soln.A |
| 7 | E | A | A | E | same as test 6 |

The data in Table III clearly demonstrates that ophthalmic solutions with pH as high as 9.4 are comfortable in the human eye. This example is also significant in that it demonstrates the utility of including acid sensitive components such as sodium bicarbonates in ophthalmic solutions intended for use directly in the eye.

## Claims

1. A preserved ophthalmic aqueous solution comprising:
(a) a source of hydrogen peroxide in an amount sufficient to provide hydrogen peroxide in an amount from 2 ppm to 100 ppm ; and
(b) one or more hydrogen peroxide stabilizers selected from the group consisting of
(a) compounds of the formula 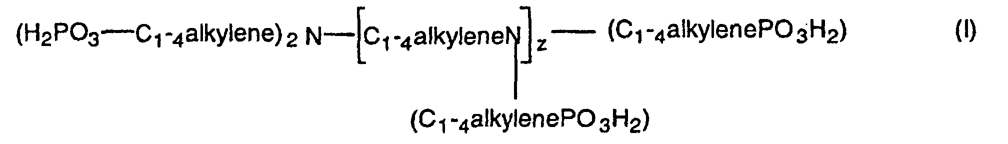 wherein z is an integer from 0-3, and water-soluble salts thereof; and
(b) compounds of the formula 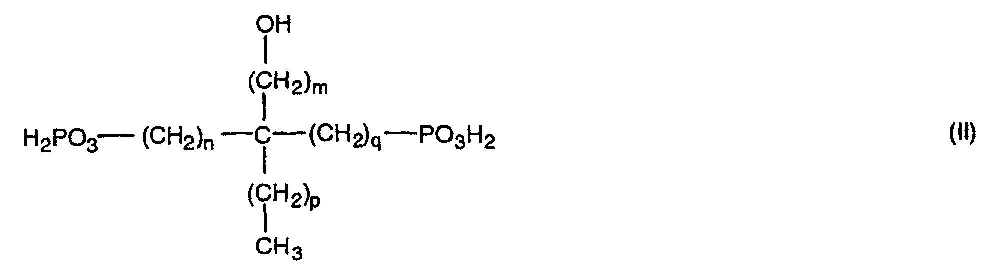 wherein each of n, m, p, and q is independently 0-4, and water-soluble salts thereof, in an amount sufficient to stabilize the resultant hydrogen peroxide; and
(c) said aqueous solution having a pH of between above 8.0 and 10.5.

2. The preserved aqueous solution of claim 1, wherein said aqueous solution has a pH of between above 8.0 and 9.5.

3. The preserved aqueous solution of claim 1 or 2, wherein said source of hydrogen peroxide is selected from the group consisting of hydrogen peroxide, sodium perborate, sodium peroxide or urea peroxide.

4. The preserved aqueous solution of any one of claims 1 to 3, wherein said source of hydrogen peroxide is sodium perborate.

5. The preserved aqueous solution of any one of claims 1 to 4, wherein in formula I, z is 2 and each of C₁₋₄ alkylene is C₁ or C₂; and wherein in formula II each of n, m, p and q is zero or 1.

6. The preserved aqueous solution of any one of claims 1 to 3, wherein said hydrogen peroxide source is selected from the group consisting of hydrogen peroxide, sodium perborate, sodium peroxide and urea peroxide, and a said hydrogen peroxide stabilizer is diethylene triamine penta(methylenephosphonic acid) or 1-hydroxyethylidene-1,1-diphosphonic acid, or a water-soluble salt thereof.

7. The preserved aqueous solution of claim 6, wherein the amount of diethylene triamine penta(methylenephosphonic acid) or water-soluble salt thereof, is from 0.002% to 0.03% by weight, and said effective amount of 1-hydroxyethylidene-1,1-diphosphonic acid or water-soluble salt thereof is from 0.005% to 0.2% by weight.

8. A preserved ophthalmic solution according to claim 7, wherein the source of hydrogen peroxide is sodium perborate and the hydrogen peroxide stabilizer is diethylene triamine penta(methylenephosphonic acid).

9. A preserved ophthalmic drug formulation comprising:
(a) an effective amount of an ophthalmic medicinal agent which is compatible with hydrogen peroxide;
(b) a source of hydrogen peroxide for providing hydrogen peroxide in an amount of 2 ppm to 100 ppm; and
(c) one or more hydrogen peroxide stabilizers of claim 1 in sufficient amount to stabilize the hydrogen peroxide;
said formulation having a pH of between above 8.0 and 10.5.

10. The preserved ophthalmic drug formulation of claim 9, wherein said pH is between above 8.0 and 9.5.

11. The preserved ophthalmic drug formulation of claims 9 or 10, wherein said hydrogen peroxide source is selected from the group consisting of hydrogen peroxide, sodium perborate, sodium peroxide and urea peroxide, and a said hydrogen peroxide stabilizer is diethylene triamine penta(methylenephosphonic acid) or 1-hydroxyethylidene-1,1-diphosphonic acid, or water-soluble salts thereof.

12. The preserved ophthalmic drug formulation of claim 11, wherein said effective amount of diethylene triamine penta(methylenephosphonic acid) or water-soluble salt thereof, is from 0.002% to 0.03% by weight, and said effective amount of 1-hydroxyethylidene-1,1-diphosphonic acid or water-soluble salt thereof is from 0.005% to 0.2% by weight.

13. The preserved ophthalmic drug formulation of any one of claims 10 to 12, wherein the ophthalmic medicinal agent is hyaluronic acid or sodium hyaluronate.

14. The preserved ophthalmic drug formulation of any one of claims 9 to 13, wherein said hydrogen peroxide source is sodium perborate and a said hydrogen peroxide stabilizer is diethylene triamine penta(methylenephosphonic acid).

## Patentansprüche

1. Konservierte ophthalmische wässrige Lösung, umfassend:
(a) eine Wasserstoffperoxidquelle in einer ausreichenden Menge, um Wasserstoffperoxid in einer Menge von 2 ppm bis 100 ppm bereitzustellen; und
(b) einen oder mehrere Wasserstoffperoxidstabilisatoren, ausgewählt aus der Gruppe, bestehend aus
(a) Verbindungen der Formel 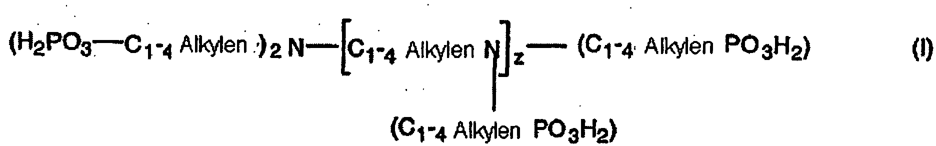 worin z eine ganze Zahl von 0 bis 3 ist und in Wasser löslichen Salzen davon; und
(b) Verbindungen der Formel 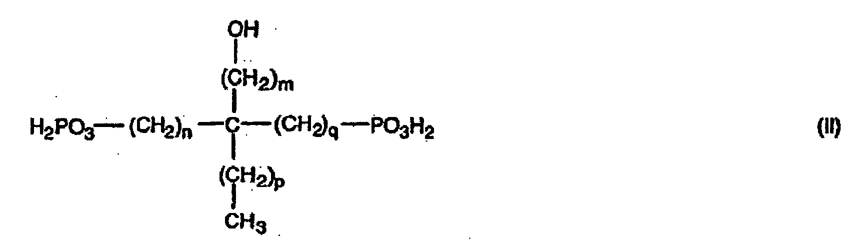 worin jeder von n, m, p und q unabhängig 0-4 ist und in Wasser löslichen Salzen davon,
in einer ausreichenden Menge, um das erhaltene Wasserstoffperoxid zu stabilisieren; und
(c) wobei die wässrige Lösung einen pH-Wert zwischen oberhalb 8,0 und 10,5 aufweist.

2. Konservierte wässrige Lösung nach Anspruch 1, worin die wässrige Lösung einen pH-Wert von zwischen oberhalb 8,0 und 9,5 aufweist.

3. Konservierte wässrige Lösung nach Anspruch 1 oder 2, worin die Wasserstoffperoxidquelle aus der Gruppe, bestehend aus Wasserstoffperoxid, Natriumperborat, Natriumperoxid oder Harnstoffperoxid, ausgewählt ist.

4. Konservierte wässrige Lösung nach einem der Ansprüche 1 bis 3, worin die Wasserstoffperoxidquelle Natriumperborat ist.

5. Konservierte wässrige Lösung nach einem der Ansprüche 1 bis 4, worin in Formel I z 2 ist und jeder von C₁₋₄-Alkylen C₁ oder C₂ ist und worin in Formel II jeder von n, m, p und q 0 oder 1 ist.

6. Konservierte wässrige Lösung nach einem der Ansprüche 1 bis 3, worin die Wasserstoffperoxidquelle aus der Gruppe, bestehend aus Wasserstoffperoxid, Natriumperborat, Natriumperoxid und Harnstoffperoxid, ausgewählt ist, und der Wasserstoffperoxidstabilisator Diethylentriaminpenta(methylenphosphonsäure) oder 1-Hydroxyethyliden-1,1-diphosphonsäure oder ein in Wasser lösliches Salz davon ist.

7. Konservierte wässrige Lösung nach Anspruch 6, worin die Menge an Diethylentriaminpenta(methylenphosphonsäure) oder in Wasser löslichem Salz davon 0,002% bis 0,03 Gewichtsprozent ist und die wirksame Menge an 1-Hydroxyethyliden-1,1-diphosphonsäure oder in Wasser löslichem Salz davon 0,005% bis 0,2 Gewichtsprozent ist.

8. Konservierte ophthalmische Lösung nach Anspruch 7, worin die Wasserstoffperoxidquelle Natriumperborat ist und der Wasserstoffperoxidstabilisator Diethylentriaminpenta(methylenphosphonsäure) ist.

9. Konservierte ophthalmische Arzneistoffformulierung, umfassend:
(a) eine wirksame Menge eines ophthalmischen medizinischen Mittels, das mit Wasserstoffperoxid verträglich ist;
(b) eine Wasserstoffperoxidquelle zum Bereitstellen von Wasserstoffperoxid in einer Menge von 2 ppm bis 100 ppm; und
(c) einen oder mehrere Wasserstoffperoxidstabilisatoren nach Anspruch 1 in ausreichender Menge, um das Wasserstoffperoxid zu stabilisieren;
wobei die Formulierung einen pH-Wert zwischen oberhalb 8,0 und 10,5 aufweist.

10. Konservierte ophthalmische Arzneistoffformulierung nach Anspruch 9, worin der pH-Wert zwischen oberhalb 8,0 und 9,5 liegt.

11. Konservierte ophthalmische Arzneistoffformulierung nach Ansprüchen 9 oder 10, worin die Wasserstoffperoxidquelle aus der Gruppe, bestehend aus Wasserstoffperoxid, Natriumperborat, Natriumperoxid und Harnstoffperoxid, ausgewählt ist und der Wasserstoffperoxidstabilisator Diethylentriaminpenta(methylenphosphonsäure) oder 1-Hydroxyethyliden-1,1-diphosphonsäure oder in Wasser lösliche Salze davon darstellt.

12. Konservierte ophthalmische Arzneistoffformulierung nach Anspruch 11, worin die wirksame Menge an Diethylentriaminpenta(methylenphosphonsäure) oder in Wasser löslichem Salz davon 0,002% bis 0,03 Gewichtsprozent ist und die wirksame Menge an 1-Hydroxyethyliden-1,1-diphosphonsäure oder einem in Wasser löslichem Salz davon 0,005% bis 0,2 Gewichtsprozent ist.

13. Konservierte ophthalmische Arzneistoffformulierung nach einem der Ansprüche 10 bis 12, wobei der ophthalmische medizinische Stoff Hyaluronsäure oder Natriumhyaluronat ist.

14. Konservierte ophthalmische Arzneistoffformulierung nach einem der Ansprüche 9 bis 13, wobei die Wasserstoffperoxidquelle Natriumperborat ist und ein Wasserstoffperoxidstabilisator Diethylentriaminpenta(methylenphosphonsäure) ist.

## Revendications

1. Solution aqueuse ophtalmique conservée, comprenant :
(a) une source de peroxyde d'hydrogène en quantité suffisante pour fournir du peroxyde d'hydrogène en une quantité de 2 ppm à 100 ppm ; et
(b) un ou plusieurs stabilisants de peroxyde d'hydrogène choisi(s) dans le groupe constitué par :
(a) des composés de formule : 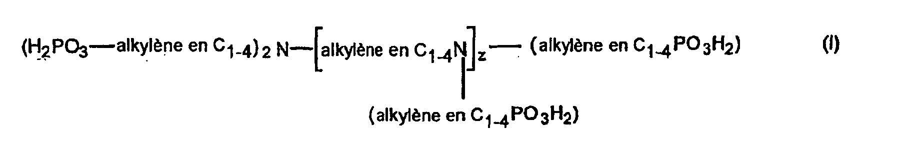 dans laquelle z est un nombre entier de 0 à 3, et des sels hydrosolubles de ceux-ci ; et
(b) des composés de formule : 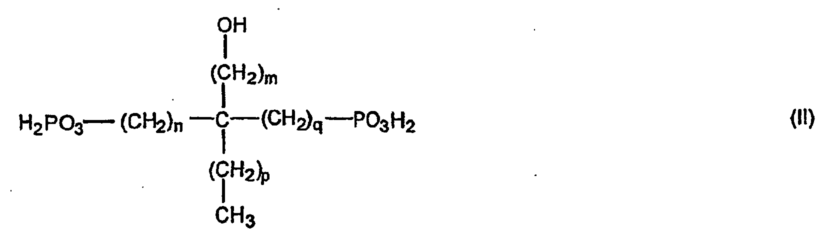 dans laquelle chacun des radicaux n, m, p et q a indépendamment une valeur de 0 à 4, et des sels hydrosolubles de ceux-ci ; en quantité suffisante pour stabiliser le peroxyde d'hydrogène résultant ; et
(c) ladite solution aqueuse ayant un pH compris entre plus de 8,0 et 10,5.

2. Solution aqueuse conservée selon la revendication 1, dans laquelle ladite solution aqueuse a un pH compris entre plus de 8,0 et 9,5.

3. Solution aqueuse conservée selon la revendication 1 ou 2, dans laquelle ladite source de peroxyde d'hydrogène est choisie dans le groupe constitué par le peroxyde d'hydrogène, le perborate de sodium, le peroxyde de sodium et le peroxyde d'urée.

4. Solution aqueuse conservée selon l'une quelconque des revendications 1 à 3, dans laquelle ladite source de peroxyde d'hydrogène est le perborate de sodium.

5. Solution aqueuse conservée selon l'une quelconque des revendications 1 à 4, où, la formule I, z vaut 2 et chaque groupe alkylène en C₁ à C₄ est en C₁ ou C₂ ; et où, la formule II, chacun des radicaux n, m, p et q vaut zéro ou 1.

6. Solution aqueuse conservée selon l'une quelconque des revendications 1 à 3, dans laquelle ladite source de peroxyde d'hydrogène est choisie dans le groupe constitué par le peroxyde d'hydrogène, le perborate de sodium, le peroxyde de sodium et le peroxyde d'urée, et ledit stabilisant de peroxyde d'hydrogène est l'acide diéthylènetriaminepenta(méthylènephosphonique) ou l'acide 1-hydroxyéthylidène-1,1-diphosphonique, ou un de leurs sels hydrosolubles.

7. Solution aqueuse conservée selon la revendication 6, dans laquelle la quantité d'acide diéthylènetriaminepenta(méthylènephosphonique) ou d'un de ses sels hydrosolubles est de 0,002 % à 0,03 % en poids, et ladite quantité efficace d'acide 1-hydroxyéthylidène-1,1-diphosphonique ou d'un de ses sels hydrosolubles est de 0,005 % à 0,2 % en poids.

8. Solution ophtalmique conservée selon la revendication 7, dans laquelle la source de peroxyde d'hydrogène est le perborate de sodium et le stabilisant de peroxyde d'hydrogène est l'acide diéthylènetriaminepenta(méthylènephosphonique).

9. Formulation d'un médicament ophtalmique conservé comprenant :
(a) une quantité efficace d'un agent médicinal ophtalmique qui est compatible avec le peroxyde d'hydrogène ;
(b) une source de peroxyde d'hydrogène pour fournir du peroxyde d'hydrogène en une quantité de 2 ppm à 100 ppm ; et
(c) un ou plusieurs stabilisants de peroxyde d'hydrogène selon la revendication 1, en quantité suffisante pour stabiliser le peroxyde d'hydrogène ;
ladite formulation ayant un pH compris entre plus de 8,0 et 10,5.

10. Formulation d'un médicament ophtalmique conservé selon la revendication 9, dans laquelle ledit pH est compris entre plus de 8,0 et 9,5.

11. Formulation d'un médicament ophtalmique conservé selon la revendication 9 ou 10, dans laquelle ladite source de peroxyde d'hydrogène est choisie dans le groupe constitué par le peroxyde d'hydrogène, le perborate de sodium, le peroxyde de sodium et le peroxyde d'urée, et ledit stabilisant de peroxyde d'hydrogène est l'acide diéthylènetriaminepenta(méthylènephosphonique) ou l'acide 1-hydroxyéthylidène-1,1-diphosphonique, ou leurs sels hydrosolubles.

12. Formulation d'un médicament ophtalmique conservé selon la revendication 11, dans laquelle ladite quantité efficace d'acide diéthylènetriaminepenta(méthylènephosphonique) ou d'un de ses sels hydrosolubles est de 0,002 % à 0,03 % en poids, et ladite quantité efficace d'acide 1-hydroxyéthylidène-1,1-diphosphonique ou d'un de ses sels hydrosolubles est de 0,005 % à 0,2 % en poids.

13. Formulation d'un médicament ophtalmique conservé selon l'une quelconque des revendications 10 à 12, dans laquelle l'agent médicinal ophtalmique est l'acide hyaluronique ou l'hyaluronate de sodium.

14. Formulation d'un médicament ophtalmique conservé selon l'une quelconque des revendications 9 à 13, dans laquelle ladite source de peroxyde d'hydrogène est le perborate de sodium et ledit stabilisant de peroxyde d'hydrogène est l'acide diéthylènetriaminepenta(méthylènephosphonique).
